# EUROPEAN PATENT APPLICATION

(11) **EP 0 696 006 A2**
(43) Date of publication of application: **07.02.1996**
(21) Application number: 95305295.8
(22) Date of filing: 28.07.1995
(51) Int. Cl.: G06F 17/60

(54) **Automatic patient reception system for hospitals**

(30) Priority: 04.08.1994 JP 204327/94; 08.12.1994 JP 31193/94
(71) Applicant: Sugiyama, Osamu, Kawagoe-shi, Saitama-ken (JP)
(72) Inventor: Sugiyama, Osamu, Kawagoe-shi, Saitama-ken (JP)
(74) Representative: Wells, David

(57) **Abstract**

An automatic patient reception system comprises a data reader (1) that converts a health insurance card or a patient ID ticket into an image data, character recognition means (3) that converts a specifically designated region of the image data into a character data, an external storage (6) that stores the patient reception data, and reception data processing means (5) that issues a reception ticket showing the order of reception and expected time of examination and treatment based on the data retrieved from the storage (6) through a printer. The order of reception printed on the reception ticket is decided based on the data stored in the external storage (6) that is retrieved by use of the data read by the data reader (1) from the health insurance card or the patient ID ticket or card.

## Description

### Field of the Invention

This invention relates to an automatic patient reception system for hospitals that prepares documents necessary for the reception of patients and other clerical work at hospitals based on patients' health insurance cards or patient ID tickets and checks the validity of patients' health insurance cards by consulting the preliminarily stored data about them.

### Description of the Prior Art

When accepting a new patient, clerical stuff of a hospital prepares an reception ticket based on the data obtained from the health insurance card of the patient and/or the hearing of his condition.

On the other hand, regular patients can complete their reception procedures even before the start of the business hours of the hospital by allowing a re-visitor reception machine to read their reception tickets issued before. As a consequence, new patients are often left unattended until the examination and/or treatment of regular patients is over. This dubiousness in the order of reception has often caused troubles between patients and hospitals.

With patients who receive medical treatment over a long period of time, it is necessary to check the validity of their health insurance cards at given intervals.

Conventionally, this validity check has been made by the clerical staff of hospitals who directly examine the health insurance cards and clinical record sheets of individual patients. However, this recheck work, which is usually done at the beginning of each month, has involved some problems, such as the heavy concentration of work load on a few specific days of the month that tends to induce various errors and mistakes.

An object of this invention is to provide an automatic patient reception system for hospitals that issues a reception ticket to both new and regular patients.

Another object of this invention is to provide an automatic patient reception system that checks the validity of health insurance cards by means of a simple processing and storage devices, without requiring human assistance.

### Summary of the Invention

An automatic patient reception system according to this invention comprises a data reader converting data on a health insurance card or a patient ID card into an image data, character recognition means converting a specific region of the image data into a character data, storage means storing patient reception data, and reception data processing means that fixes a data showing at least the order of reception based on the data retrieved from the storage means and issues a reception ticket through a printer.

When a patient, irrespective of whether he or she is a newcomer or a regular patient, places a health insurance card or patient ID card on the data reader in the reception room of a hospital, the automatic patient reception system decides the order of reception based on the data stored in the storage means and issues a reception ticket. This eliminates conventional troubles between patients and between patients and hospital staff that have arisen from the dubiousness in the order of reception.

### Brief Description of the Drawings

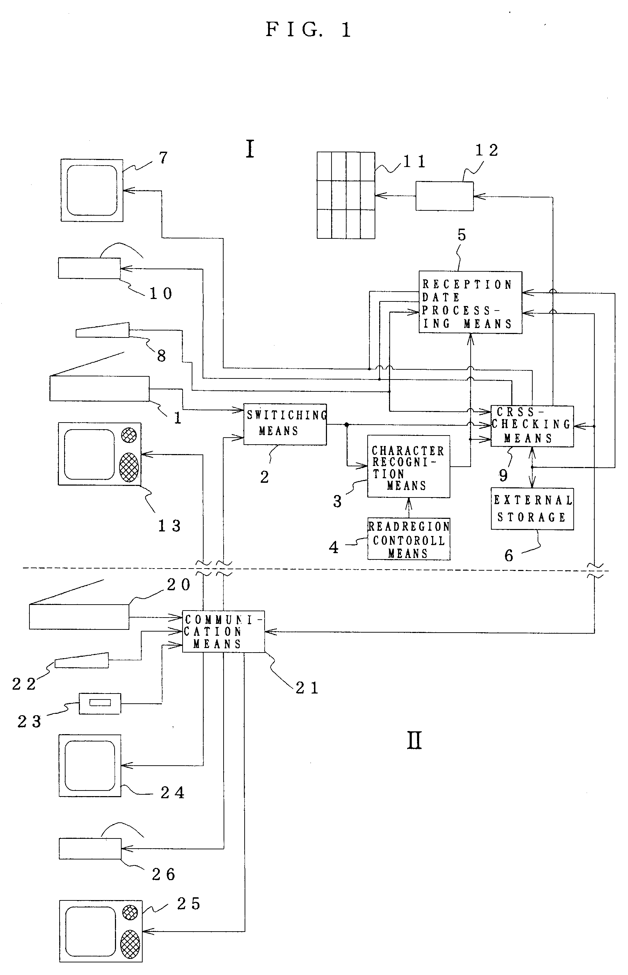

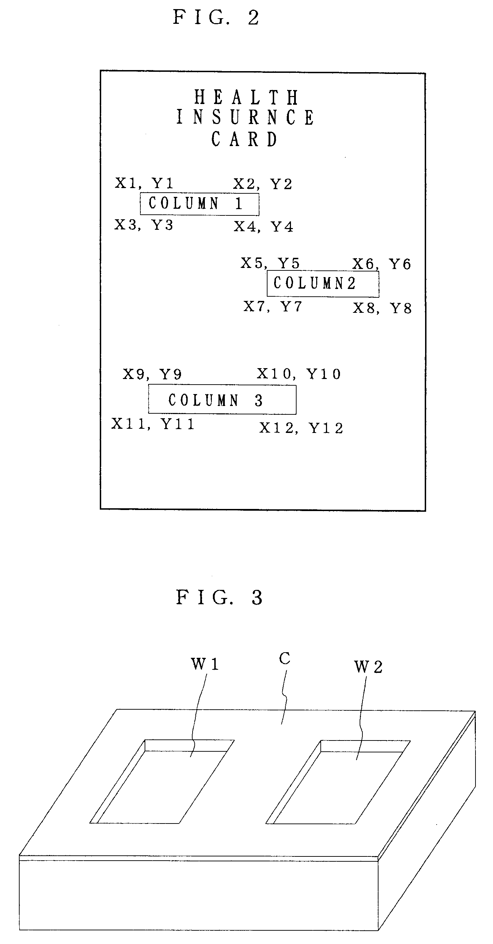

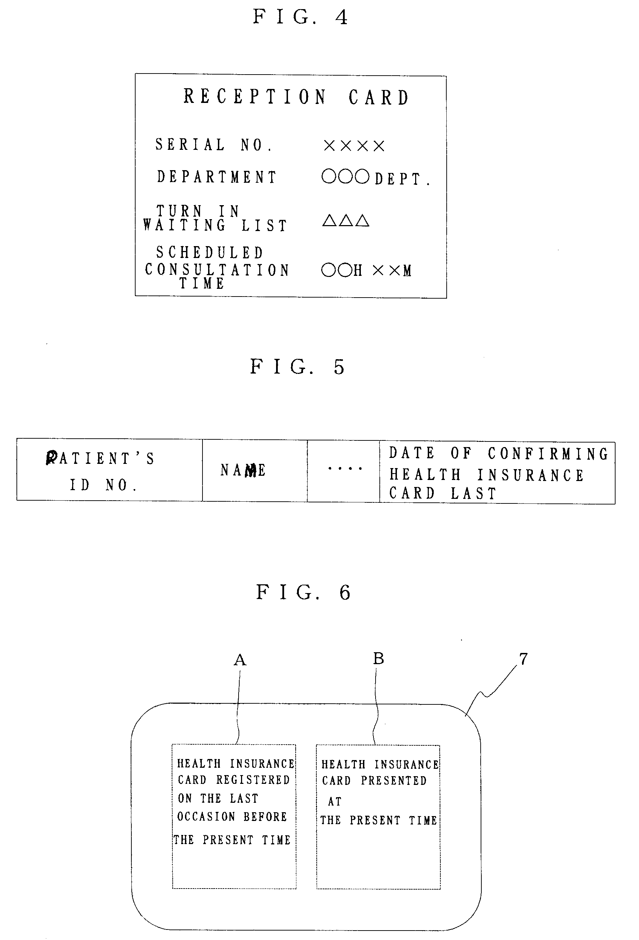

Fig. 1 is a block diagram showing an example of an automatic patient reception system according to this invention. Fig. 2 schematically shows an example of a control data on the read region control means of the same system. Fig. 3 shows an example of the data mount table of the data reader. Fig. 4 shows an example of a reception card. Fig. 5 schematically shows an example of a patient master file. Fig. 5 shows an example of an image shown on the display of the system for the verification of a health insurance card.

### Description of Preferred Embodiments

Fig. 1 shows an example of an automatic patient reception system according to this invention. Reference character I designates a system provided in the hospital staff office, whereas reference character II denotes a system provided in the reception room.

The system I in the hospital staff office will be described first.

Reference numeral 1 designates a data reader like a scanner that converts character patterns on a health insurance card or a patient ID card into a bit-mapped data, while reference numeral 2 denotes switching means that selects one of the image data output from the data reader 1 and another data reader 20 in the system II that is placed in the reception room described later.

Reference numeral 3 designates character recognition means that stores character pattern data comprising bit-mapped character patterns and corresponding character codes and outputs character codes for Japanese Kana and Kanji, and alphanumeric characters based on the bit-mapped data making up the image data from the data reader 1 or 20. Reference numeral 4 denotes read region controlling means that registers specific regions of the bit-mapped data that must be converted into character codes, such as the columns for the health insurance card number, validity, employer number and verification date. The read region controlling means then controls the character recognition means 3 to convert the bit-mapped data in the registered columns into character codes. Limiting the read regions permits quick conversion of necessary data for the verification of the patient name and health insurance card into character codes.

The read regions can be defined by pre-storing the coordinates data of each corner of the columns to be read, such as (X1, Y1), (X2, Y2), (X3, Y3) and (X4, Y4) for column 1, (X5, Y5), (X6, Y6), (X7, Y7) and (X8, Y8) for column 2, (X9, Y9), (X10, Y10), (X11, Y11) and (X12, Y12) for column 3, and so on, which are established with respect to the reference point, such as the lower right end, of the data mount table of the data readers 1 and 20, as shown in Fig. 2. Data on all health insurance cards can be converted into character codes if multiple sets of data covering all kinds of health insurance cards used at each hospital are prepared.

For a patient having two different kinds of health insurance cards, the data readers 1 and 20 have windows W1 and W2 each of which is large enough to place a health insurance card or a patient ID card thereon, as shown in Fig. 3. Two different kinds of health insurance cards can be read simultaneously when they are placed on the windows W1 and W2 into which the read region is divided into two sectors.

Reference numeral 5 designates a reception data processing means comprising a personal computer, work station or the like, to which the character recognition means 3, a card reader 23, an external mass storage 6 such as a hard disk or a magneto-optical disk, a display 7, a keyboard 8 and other peripherals are connected. The reception data processing means 5 is programmed to determine the serial number, department, order and expected time of examination and treatment for each patient, irrespective of whether the patient is a new one or a regular one, on the basis of the reception data contained in a reception master file stored in the external storage 8, the health insurance and patient ID cards placed on the data reader 20, and data read from a patient ID card inserted in the card reader 23. The reception data processing means 5 further issues a reception ticket of the type shown in Fig. 4 through a printer 26 in the reception room II, determines the identification number for a new patient as a proof of identity, and stores the assigned identification number, name, date on which the validity of the health insurance card was checked last time and other necessary data in a master file for each patient stored in the external storage 6 as shown in Fig. 5.

Particularly when the patient is a new one, the reception data processing means 5 converts the necessary data read from the health insurance card into a character data that is then used as the data for issuing the reception ticket mentioned before. The external storage 6 stores an image or bit-mapped data of the entirety of the health insurance card, which is related to the ID number, received through cross-checking means 9 described later.

The data required for the issuance of the reception ticket for a new patient can be obtained by determining whether the patient is the beneficiary of the health insurance or a family member thereof and, when necessary, the name of the department where the patient wishes to have a medical examination and treatment using a keyboard 22. The expected time of examination and treatment can be easily derived by determining the mean time spent for the examination and treatment of one patient based on the experience of each hospital. The mean time thus determined can be used as the lag time for each waiting patient at each department.

Reference numeral 9 designates cross-checking means comprising a personal computer, work station or the like, to which the external mass storage 6 such as a hard disk or a magneto-optical disk, display 7, keyboard 8 and other peripherals are connected. In addition to serving as an ordinary cross-checking device, the cross-checking means 9 also facilitates the comparison and check of health insurance cards for their verification by simultaneously showing an image data retrieved from the external storage 6 using a predetermined variable and an image data obtained from the data reader 20 described later in the two zones A and B into which the display area of the display 7 is bisected as shown in Fig. 6.

In this embodiment, a printer 10 that outputs hard copies of retrieved data and a file retriever 12 of the type, for example, disclosed in Japanese Provisional Patent Publication No. H5-189489 (1993) that retrieves desired files from a file shelf 11 are also connected. The external storage 6 has a storage region to store the reception master file mentioned before and such data as the serial number, validity time and verification date of health insurance cards, and employer numbers using all or part, such as the lower four digits, of the character codes read by the character recognition means as character variables and a storage region to store the image data of the health insurance cards read by the data reader 1.

Now, the system II in the reception room will be described.

Reference numeral 20 designates a data reader having windows W1 and W2, as shown in Fig. 3, to place the health insurance and patient ID cards therein, which makes the handling of the data reader easier for ordinary patients and other untrained people. The data reader 20 is connected to the switching means 2 in the hospital staff office I through communication means 21.

Reference numerals 22, 23, 24 and 25 respectively denote a keyboard, a card reader to read the patient ID number from the patient ID card or other cards of patients, a display, and an intercom 25 to give instructions to those who are unaccustomed to the use of the data reader 20. Such instructions are given, for example, by showing the contents of a help file stored in the external storage 6 on the display 24 when a certain pre-programmed key is pressed. The intercom 25 is connected to another intercom 13 in the hospital staff office I through communication means 21. Equipped with a display, microphone and speaker, the intercom 25 gives instructions to patients while showing images as required, and answers questions brought up by patients. Reference numeral 26 denotes a printer in the reception room II that outputs hard copies of the data retrieved from the external storage 6 in the hospital staff office I and issues the reception tickets mentioned earlier.

The following paragraphs describe the operation of the systems described above.

On arriving at a hospital, a new patient places a health insurance card and a regular patient having a patient ID ticket places the patient ID ticket in the windows W1 or W2 of the data reader 20 in the reception room II. A regular patient having a patient ID card places the patient ID card in the card reader 23.

The character recognition means 3 converts the necessary parts of the health insurance or patient ID card placed on the data reader 20 into a bit-mapped data in character codes that are processed by the reception data processing means 5. When the patient specifies whether he or she is the beneficiary of the health insurance or a family member thereof and the name of the department where the patient wishes to have a medical examination and treatment using a keyboard 22, the reception data processing means 5 determines the serial number, department, order and expected time of examination and treatment based on the reception master file in the external storage 6 and issues a reception ticket from the printer 22 in the reception room II. The reception master file comprises a reception order file and an expected examination time file that distinguish between the new and regular patients.

When a reception data is input from the character recognition means 3 or card reader 23, the reception order file is opened to determine the serial number while distinguishing between new and regular patients and updating the contents of the file. Then, the expected examination time file is opened to determine the expected examination time of each department by adding the time lags mentioned earlier. The order of examination and treatment for each patient is derived from the time thus determined, and the contents of the expected examination time file is updated. When a regular patient inserts his or her patient ID card into the card reader 23, the reception data processing means 5 issues a reception ticket based on the data read therefrom.

Because the reception tickets are issued to all patients, irrespective of whether they are on a first visit or revisit, the order of examination and treatment for each patient is definitely fixed. As patients are aware of, in addition, approximate examination time, they can make efficient use of their waiting time without being confined in the waiting room of the hospital. This, in turn, eliminates the need to prepare unnecessarily large waiting rooms.

In the case of a new patient, the character recognition means 3 further converts data in specific regions, such as the columns describing the insurance card number, validity, or employer number, into character codes. Then, a patient ID card carrying, for example, the lower four digits of the health insurance card number, such as "4567" of "1234567", as the patient's ID number is issued. The patients are required to present the patient ID card whenever they make a revisit. When the character variable "4567" is input after the new registration command from the keyboard 8, the insurance card number, validity, employer number from the character recognition means 3 and the image data of the health insurance card read by the data reader 1 are stored in the patient's master file in the external storage 6.

In the case of a patient on a revisit, the card reader 23 reads his or her ID number from the patient ID card. Then the ID number is sent through the communication means 21 to the cross-checking means 9 as a character string for retrieval. Using the ID number, the cross-checking means 9 retrieves the file of the patient from the external storage 6, finds the date when the validity of the health insurance card was verified on the last occasion, and checks if the validity has been verified in the current month. If it is found that the validity of the health insurance card has already been verified in the current month, the cross-checking means 9 outputs the ID number to the file retriever 12 for automatic selection of the clinical record and other documents necessary for the examination and treatment from the file shelf 11.

When it is found that the validity of the health insurance card has not been verified in the current month, the display 7 shows the image data of the health insurance card retrieved from the external storage 6 using the ID number in the left half thereof (region A in Fig. 6). At the same time, the intercom 25, which is placed next to the card reader 23 at the reception desk so as to be readily noticeable to the patient standing in front of the card reader, shows a message "Place the health insurance card on the data reader 20 for input of the necessary data." on the display thereof while giving audio instructions.

When the patient places the health insurance card on the data reader 20 in conformity with the given instructions, the image data sent therefrom through the communication means 21 is shown in the other half of the display 7 (region B in Fig. 6) in the hospital staff office I. A staff member at the display 7 can visually confirm the validity of the health insurance card by comparing the new data in region B with the old one in region A, without taking the trouble of seeing the health insurance card it-self.

At the same time, the character recognition means 3 converts the numbers described in the specific part of the image data of the health insurance card placed on the data reader 20 into character codes that are input to the cross-checking means 9 for checking if the specific character string preselected for each health insurance card is contained therein.

When the new data agrees with the registered one, the validity of the health insurance card is verified. Then, the date of verification in the external storage 6 is updated, while the ID number is output to the file retriever 12 for selection of necessary documents. The intercom 25 then announces the completion of the reception procedure and gives instructions that the patient should wait in front of the examination room of the desired department.

When the two data do not agree with each other, instructions are given to the staff member in the office to take necessary steps. When the two image data shown on the display 7 are found to disagree, there is a likelihood that the patient is on the first visit or that the health insurance card has been renewed. Then, the staff member takes necessary steps for the reception of a new patient by sending a new patient reception command to the cross-checking means 9 from the keyboard 8.

Verification of the health insurance card is usually done when the patient visits the hospital for the first time in the month. Reception is not given until the automatic verification of the health insurance card is complete. When the number of patients is large, the number of health insurance cards requiring verification too becomes so great as to impede the processing of other work. The patients who make their first visit will be requested to present their health insurance cards again in much shorter periods of time than the basic intervals.

The system of this invention is programmed to leave a period of 30 days for new patients between the first date of verification and the next. For regular patients, the program widens the time intervals by some days when the frequency of visits is high and keeps the basic intervals when the frequency of visits is low.

This program frees patients from extra procedural loads and averages the work load for the verification of health insurance cards throughout the month. Specific numerals described in the specifically designated part of the health insurance cards of new patients are treated as a character variable making up a variable-length data that is added to a file of previously registered data. The character variable consists of, for example, 10,000 arrays that can be expressed as DIMA$ (10000). While part, e.g. four digits, of the health insurance card number is used as the patient ID number on the patient ID ticket or card, full digits thereof are used as the ID number of the data file of the same patient stored in the external storage.

When a health insurance card has, for example, a serial number "1234567," the lower four digits thereof are used as the patient ID number on the ID ticket and card. A character variable A$(4567) based on the ID number thus given also contains the remaining numerals making up the serial number (i.e., 123) and other numerals described in other specifically designated parts of the health insurance card, such as the date when the validity of the card was verified. When the character variable A$(4567) is read out, for example, a character string "123; 878787; 11/30" appears. In this character string, "123" is the remaining numerals of the serial health insurance card number, "878787" is the specific numerals described in the specifically designated part of the card, and "11/30" represents the 30th day of November on which the card was verified.

Of course, the character variable A$(4567) can also contain some other data, such as the employer number. When a patient whose ID card number is "4567" revisits the hospital on the 5th day of December, the verification date is automatically updated from "11/30" to "12/05" upon presentation of the health insurance card to the hospital office in conformity with the procedures mentioned earlier.

In the embodiment described above, cross-check is made by using part of the health insurance card number or the data file ID number. As a result, another different ID number having the same lower four digits, such as "994-567", can also be stored as a character variable A$(4567). If data file ID numbers "1234567" and "994567" are registered on November 30th and December 1st, two character strings, i.e., "123; 878787; 11/30" and "99; 565656; 12/01" will appear when A$(4567) is called up. When the number of data files exceeds the limit set for one ID number, the overflowing data files are stored under another character variable, such as B$(1000). This permits recording as many data files as desired.

The embodiment described above handles data as character variables. If the external storage 6 has a large enough storage capacity, data files numbered, for example, "1234567" and "994567" and their subordinate data can be stored in a single file numbered "4567". When the file "4567" is called out, all data contained therein are retrieved for use.

The embodiment described above decides the order of examination and treatment without separating new patients from regular ones. Obviously, however, some clinics and hospitals may provide separate receptions for new and regular patients instead of a unified one. Under such a separate reception system, a patient on the first visit will receive a reception card marked "First Visit" and treated separately in such a way as to fit the circumstances of individual clinics and hospitals.

## Claims

1. An automatic patient reception system for hospitals characterised by a data reader (1) for converting a health insurance card or a patient ID ticket into an image data, character recognition means (3) for converting a specifically designated zone of the image data into a character data, storage means (6) for storing patient reception data, and reception data processing means (5) for issuing a reception ticket from a printer based on the data indicating at least the order of reception that is prepared based on the data retrieved from said storage means (6).

2. An automatic patient reception system according to claim 1 that prints the order and expected time of examination and treatment on the reception ticket.

3. An automatic patient reception system according to claim 1, in which the data reader (1) has multiple regions where multiple health insurance cards or patient ID tickets are placed and read.

4. An automatic patient reception system according to any one of the preceding claims, further comprising a card reader for electrically, magnetically or optically reading the ID number of a patient from a patient ID card inserted therein.

5. An automatic patient reception system according to claim 4, further characterised in that the storage means (6) are arranged to store the image data from the data reader (1) and the character data in such a way as to be retrievable by using the patient ID number, and by cross-checking means (9) for retrieving a desired data from the storage means (6) and performing necessary verification on output of the patient ID number from the card reader.

6. An automatic patient reception system according to claim 5, in which the desired data is the validity or the date of previous verification of a health insurance card.

7. An automatic patient reception system according to claim 5 or 6, in which the intervals at which the desired data is verified are variable.

8. An automatic patient reception system according to claim 5, 6 or 7 in which the card reader and a second data reader are placed in a reception room, and the cross-checking means shows the image data from the storage means and the second data reader on separate regions of one and the same display.

9. An automatic patient reception system according to claim 8, in which communication means to support the function of the second data reader is provided adjacent to the second data reader.
